# EUROPEAN PATENT SPECIFICATION

(11) **EP 2 138 049 B1**
(45) Date of publication and mention of the grant of the patent: **25.06.2014**
(21) Application number: 08739646.1
(22) Date of filing: 26.03.2008
(51) Int. Cl.: A23L 1/00, A23L 1/317, C12N 9/10, A23L 1/0562, A23J 3/22, A23J 3/34

(54) **ENZYME PREPARATION FOR ADHESION AND METHOD OF PRODUCING ADHESION-MOLDED FOOD PRODUCT**
ENZYMHERSTELLUNG FÜR ADHÄSION UND VERFAHREN ZUR HERSTELLUNG EINES ADHÄSIONSGEFORMTEN LEBENSMITTELPRODUKTS
PRÉPARATION ENZYMATIQUE POUR ADHÉRENCE ET PROCÉDÉ DE PRODUCTION D'UN PRODUIT ALIMENTAIRE MOULÉ PAR ADHÉRENCE

(30) Priority: 29.03.2007 JP 2007086631
(43) Date of publication of application: 30.12.2009
(73) Proprietor: Ajinomoto Co., Inc., Tokyo 104-8315 (JP)
(72) Inventor: ISHIDA, Rikiya, Kawasaki-shi Kanagawa 210-8681 (JP); OGAWA, Teppei, Kawasaki-shi Kanagawa 210-8681 (JP)
(74) Representative: Strehl Schübel-Hopf & Partner
(86) International application number: PCT/JP2008/056535
(87) International publication number: WO 2008/120798

(56) References cited:
- EP-A1- 1 374 699
- EP-A1- 1 543 732
- EP-A2- 0 815 742
- WO-A1-97/40701
- WO-A1-02/080700
- JP-A- 10 070 961
- JP-A- 2006 246 716
- US-A- 4 917 904
- KUTEMEYER C ET AL: "The influence of salts and temperature on enzymatic activity of microbial transglutaminase", FOOD CONTROL, BUTTERWORTH, LONDON, GB, vol. 16, no. 8, 1 October 2005 (2005-10-01), pages 735-737, XP004776616, ISSN: 0956-7135, DOI: 10.1016/J.FOODCONT.2004.06.012
- SAKAMOTO H ET AL: "STRENGTH OF PROTEIN GELS PREPARED WITH MICROBIAL TRANSGLUTAMINASE AS RELATED TO REACTION CONDITIONS", JOURNAL OF FOOD SCIENCE, WILEY-BLACKWELL PUBLISHING, INC, US, vol. 4, no. 59, 1 January 1994 (1994-01-01), pages 866-871, XP001070307, ISSN: 0022-1147, DOI: 10.1111/J.1365-2621.1994.TB08146.X
- FERNANDEZ-DIAZ M.D. ETAL.: 'Gel properties of collagens from skins of cod (Gadus Morhua) and hake (Merluccius merluccius) and their modification by the coenhancers magnesium sulphate glycerol and transglutaminase' FOOD CHEMISTRY vol. 74, 2001, pages 161 - 167, XP008115964
- CHEN R.-N. ET AL.: 'Characterization of collagen matrices crosslinked using microbial transglutaminase' BIOMATERIALS vol. 26, 2005, pages 4229 - 4235, XP004729337

## Description

### Technical Field

This invention relates to an enzyme preparation for binding and a production method for a bound and formed food.

### Background Art

Many reports have been made on a technology for binding and forming a food material using a transglutaminase.

Japanese Patent No. 1927253 discloses a technology for producing a bound and formed food with the use only of a transglutaminase. The invention was a breakthrough technology that developed a new utility of transglutaminase, but, due to the lack of capability of attaining a sufficient binding strength, many studies were conducted on combined use with various components to put the technology into practical use.

Also, each of Japanese Patent No. 3353383 and Japanese Patent No. 3353503 discloses an invention for a bound and formed food obtained by combining a transglutaminase and caseins used as a substrate for the transglutaminase. These methods are widely applicable to food materials including fish meats, seafood such as squid and crab, fish eggs such as salmon roe (ikura), herring roe, salmon roe (sujiko) and cod roe without limitation to animal meats. Also, since these methods enable binding a raw food, these inventions relate to a binding and forming method that does not influence taste and flavor and is highly versatile.

Meanwhile, due to recent food allergy problems, a milk-origin protein is not usable in processed food in some cases. Therefore, binding methods using a combination of a transglutaminase and a protein other than caseins have also been studied.

Japanese Patent No. 3407599 discloses a binding and forming method using collagen and a transglutaminase as active ingredients without using casein in combination. However, since the collagen possesses a property of expressing high viscosity when dissolved into water, it is essential to dissolve the collagen into cold water of 10°C or less, and it is necessary to start binding operation soon after the dissolution, thereby entailing a kind of problem in workability.

Also, since the binding strength is weak without the use of a salt, and it is difficult to expect practical effect when salt is not used.

International Publication No. WO02/080700 discloses an enzyme preparation for binding for food materials, comprising as active ingredients a specific collagen in which the number of total residues of hydroxyproline and proline (hereinafter sometimes referred to as imino acid) in collagen is less than 20% of the number of total amino acid residues in collagen and a transglutaminase and a method for producing a bound and formed food using the enzyme preparation for binding. Also, JP-A-2006-246716 discloses a method for producing a bound and formed food using a food binding agent containing a transglutaminase, collagen, a pH adjuster such as citric acid or trisodium phosphate, and calcium chloride, and it is described that "calcium chloride or the like may preferably be added since the addition of calcium chloride enables suppression of gelation of the collagen at a low temperature". However, the publication does not disclose an appropriate amount of calcium chloride to be added.

Usable as the above-mentioned specific collagen substantially' is a fish skin-derived collagen, and the preparation containing fish collagen is not usable for animal meat processed foods using pork, beef, and chicken in some cases since the fish collagen is a protein of different origin.

Particularly, in the European market, allergy food labeling of processed food using fish-derived material is obligatory, and the use of fish-derived material for animal meat processed food is more restricted in some cases as compared to other countries.

In the past, a technology for expressing practical binding strength by using collagen derived from pork, beef, or chicken has not been developed.

### Disclosure of the Invention

In view of the above-described background, there is a demand in food processing industry for an excellent enzyme preparation for binding and forming and a production method for a bound and formed food capable of satisfactorily binding and forming a food material such as pieces of meat without using a fish-derived material. Therefore, an object of this invention is to provide an excellent enzyme preparation for binding and forming and a production method for a bound and formed food.

The inventors conducted extensive research in the aim of solving the above-described problems to find that it is possible to bind a food material such as cattle meat by using a transglutaminase, collagen, and calcium chloride and/or magnesium chloride, thereby accomplishing this invention. More specifically, this invention is as described below.
1) An enzyme preparation comprising as active ingredients a transglutaminase derived from a microorganism, animal collagen not including fish and shellfish collagen, and calcium chloride or magnesium chloride, wherein an amount of calcium chloride or magnesium chloride in the enzyme preparation is 0.007 to 0.03 g per 1 unit of the transglutaminase in the enzyme preparation, and an amount of the collagen in the enzyme preparation is 0.002 to 0.03 g per 1 unit of the transglutaminase in the enzyme preparation.
2) The enzyme preparation according to 1), wherein a gel strength of the collagen comprised in the enzyme preparation is 40 g or more.
3) The enzyme preparation according to 1), wherein a jelly strength of the collagen comprised in the enzyme preparation is 150 g or more.
4) The enzyme preparation according to 3), wherein the collagen is an acid-treated collagen.
5) A method for producing a bound and formed food, wherein the enzyme preparation according to 1) to 4) is (1) dissolved into water or a liquid to be added to a food material or (2) added directly to a food material.
6) A method for producing a bound and formed food, wherein 10 to 100 units of a transglutaminase derived from a mircoorganism, 0.1 to 2 g of animal collagen not including fish and shellfish collagen, and 0.4 to 2 g of calcium chloride or magnesium chloride per 100 g of the food material are (1) dissolved into water or a liquid to be added to a food material or (2) added directly to a food material.
7) The method according to 6), wherein a gel strength of the collagen comprised in the enzyme preparation is 40 g or more.
8) The method according to 6), wherein a jelly strength of the collagen comprised in the enzyme preparation is 150 g or more.
9) The bound and formed food production method according to 8), wherein the collagen is an acid-treated collagen.

This invention will hereinafter be described in detail. This invention is characterized by causing (1) collagen and (2) calcium chloride or magnesium chloride to function as an enzyme preparation for binding in addition to an enzymatic effect of a transglutaminase.

The transglutaminase to be used in this invention is an enzyme used for catalyzing an acyl-transfer reaction in a γ-carboxyamide group in a glutamine residue in a protein or peptide chain. A ε-(γ-Glu)-Lys bond is formed in and between protein molecules by the action of the transglutaminase as an acyl receptor exerted on a ε-amino group of a lysine residue in the protein.

As the transglutaminase to be used as the enzyme in this invention, include those derived from a microorganism such as those derived from actinomycete (see Japanese Patent No. 2572716) and those derived from bacillus subtilis (see Japanese Patent No. 3873408). Also, examples of the transglutaminase include those derived from guinea pig liver (see Japanese Patent No. 1689614), those derived from a microorganism (see WO96/06931), those derived from an animal such as cow blood and pig blood; those derived from fish such as salmon and sea red bream (Seki et al.; Journal of Japanese Society of Fisheries Science; 1990; 56; 125-132); those derived from oyster (see United States Patent No. 5736356); and the like.

Also, the examples of the transglutaminase include those produced by gene recombination (for example, see Japanese Patent No. 3010589, JP-A-11-75876, WO01/23591, WO02/081694, WO2004/078973), and the like.

As described above, it is possible to use any one of the above-listed transglutaminases. From the viewpoints of functionality and handling easiness for use in foods, it is preferable to use the transglutaminase derived from microorganisms since they are mass-produced and available at a low cost (Registered No. 2572716 and the like).

An activity unit of the transglutaminase to be used in this invention is measured and defined as described below. Specifically, a reaction is performed by using benzyloxycarbonyl-L-glutaminylglycine and hydroxylamine as a substrate, and, after converting thus-generated hydroxamic acid into an iron complex in the presence of trichloroacetic acid, an amount of the iron complex is measured at an absorbance of 525 nm. An enzyme amount that enables generation of 1 micromol of hydroxamic acid in one minute is defined as 1 unit which is an activity unit of transglutaminase. Details of this measurement method which is called hydroxamate method have already been reported (for example, see Registered No. 2572716).

As described above, transglutaminase is known to have various origins. There also are some transglutaminase whose activity cannot be defined by the hydroxamate method due to substrate specificity. In such case, the unit is defined by a different method. An amount that substantially attains the binding and forming effect of this invention is within the range of the transglutaminase addition of this invention irrespective of the activity measurement method employed for definition.

Calcium chloride and magnesium chloride that have a grade usable for foods may be used in this invention, and calcium chloride and magnesium chloride may be used alone or in combination.

As the collagen that is an essential component in this invention, any type of animal collagen not including fish and shellfish collagen may be used. More specifically, materials for the collagen to be used in this invention is not particularly limited, and those extracted from tissues such as skin, bone, cartilage, scale, bladder, and the like of an animal may be used. The binding effect is considerably improved by the use of the collagen in addition to a salt described later in this specification and the transglutaminase.

As used herein, the collagen means those obtained from skin, bone, cartilage, scale, and bladder of an animal by extraction and purification, and a degree of modification such as decomposition is not limited. Since the collagen is hydrolyzed into various degrees in the course of the extraction, the collagen generally has a wide-range of molecular weight distribution, and those formed into a so-called gelatin by attaining a low molecular weight are within the range of the collagen of this invention. Further, the collagen is not necessarily be a purified substance, and fat, carbohydrate, peptide, amino acid, and the like may partially be contained therein.

Among the collagens, those obtained without undergoing an acid treatment, an alkali treatment, and the like are preferred since such collagen contributes to realization of a stronger binding strength, and it is more preferable to use the collagen having a gel strength of 40 g or more. The gel strength is measured as described below. After dissolving the collagen into 30°C warm water for about 1 hour to achieve a concentration of 5%, 50 ml of the collagen is poured into a single purpose jelly cup, and the jelly cup is sealed and cooled in a 5°C refrigerator for 17 hours. After the cooling, the gel is subjected to a measurement using a physical properties measurement device such as a texture analyzer and a rheometer by using a spherical probe having a diameter of 5 mm at an intrusion rate of 1 mm/sec and an intrusion distance of 8 mm, and the thus-detected stress value (g) is the gel strength.

In the case of using collagen subjected to the acid treatment or the alkali treatment, a jelly strength of the collagen may be 150 g or more, preferably 150 to 300 g, more preferably 250 to 300 g, since such collagen expresses a stronger binding strength and is suitably used.

The jelly strength is measured as described below. After dissolving the collagen to achieve a concentration of 6.67%, 120 ml of the collagen is poured into a single purpose jelly cup and left to cool to 35°C at a room temperature. Subsequently, a rubber cap is covered on the jelly cup, and the jelly cup is placed in an incubator of 10°C to be cooled for 17 hours. The gel after the cooling is subjected to a measurement using a physical properties measurement device such as a texture analyzer and a rheometer by using a cylindrical probe having a diameter of 12.7 mm at an intrusion rate of 1 mm/sec and an intrusion distance of 4 mm, and the thus-detected stress value (g) is the jelly strength (see JIS K 6503).

In order to realize a further stronger binding strength, it is more preferable to use the collagen that is subjected to the acid treatment during its production process as the collagen than to use the alkali-treated collagen. The acid-treated collagen and the alkali-treated collagen are different from each other in isoelectric point, and the acid-treated collagen exhibits a pH level of about 6.5 to 9.0, while the alkali-treated collagen exhibits a pH level of about 4.9 to 5.2. A test is performed by employing the PAGI method (Commission on Methods for Testing Photographic Gelatin; PAGI method; PAGI method 9th edition 2002) as the test method. In the PAGI method employed in this invention, a pH level of a solution obtained by eliminating, from a gelatin solution, ionic substances by using a resin obtained by mixing an anion exchange resin and a cation exchange resin is set as an isoelectric point.

Regarding the formulation rate of the transglutaminase, the collagen, and calcium chloride or magnesium chloride forming the enzyme preparation of this invention, 1 to 200 units of transglutaminase is added per 1 g of the enzyme preparation. 5 to 95 parts by weight of calcium chloride or magnesium chloride is added per 100 parts by weight of the enzyme preparation. 5 to 95 parts by weight of the collagen is added per 100 parts by weight of the enzyme preparation. Regarding ratios of the components, an amount of calcium chloride or magnesium chloride (a total amount of calcium chloride and magnesium chloride in the case of combined use) per 1 U of the transglutaminase in the enzyme preparation may preferably be 0.007 to 0.03 g, more preferably 0.01 to 0.02 g, and a dry weight of the collagen per 1 U of the transglutaminase in the enzyme preparation may preferably be 0. 002 to 0. 03 g, more preferably 0.003 to 0.007 g.

The enzyme preparation for binding of this invention contains as active ingredients the transglutaminase, the collagen, and calcium chloride and/or magnesium chloride, and various arbitrary components described below may be added thereto. For example, those known as a food diluent such as lactose, sucrose, maltitol, sorbitol, dextrin, branched dextrin, cyclodextrin, silicon dioxide, cellulose, a starch, polysaccharide, a gum, pectin, and the like may be added.

Further, for the enzyme preparation for binding, it is possible to use a protein other than the collagen, such as a protein extracted from an animal meat including pork and beef, a protein extracted from domestic poultry, a soy protein, a wheat protein, and a casein. Also, a partial hydrolysate of these proteins (partially hydrolyzed soy protein, partially hydrolyzed wheat protein, etc.) may be used.

Also, sodium hydrogen carbonate, sodium citrate, sodium phosphate, calcinated calcium, calcium phosphate, calcium carbonate, magnesium carbonate, sodium chloride, potassium chloride or kinds of polyphosphate like sodium pyrophosphate, sodium tripolyphosphate and sodium metaphosphate may be added to the enzyme preparation. Further, a seasoning, a sugar, a spice, a colorant, a color former, ascorbic acid and salts thereof, an emulsifier, an oil, and the like may be appropriately added without any difficulty.

As the enzyme preparation for binding and forming of this invention, the transglutaminase, the collagen, and calcium chloride or magnesium chloride are not necessarily blended in a container, and a mode of a so-called "kit" in which the transglutaminase, the collagen, and calcium chloride or magnesium chloride are independently contained in separate containers is encompassed by the enzyme preparation. The enzyme preparation may be in the form of a powder or a liquid.

In the case of producing a bound and formed food by binding a food material, the following methods are considered. Specifically, a method of using the enzyme preparation containing as active ingredients the transglutaminase, the collagen, and calcium chloride or magnesium chloride, and a method of using the transglutaminase, the collagen, and calcium chloride or magnesium chloride that are separately purchased. Either method may be used.

In each of the methods, a use amount of the transglutaminase for the food material may be 1 to 400 units, preferably 10 to 200 units, per 100 g of the food material used as the object of the binding. A use amount of the collagen per 100 g of the food material may be 0.02 to 5 g, preferably 0.1 to 2 g. A use amount of calcium chloride or magnesium chloride (a total amount of calcium chloride and magnesium chloride in the case of combined use) per 100 g of the food material may be 0.02% to 5%, preferably 0.4% to 2%.

In the case of producing a bound and formed food by binding a food material, the following methods are considered. Specifically, a case of dissolving the enzyme preparation for binding of this invention into a solvent and a case of mixing the enzyme preparation in the form of a powder directly with the food material are considered. More specifically, a case of dissolving each of the essential components such as the transglutaminase and mixing the components separately or simultaneously with the food material, and a case of using the essential components such as the transglutaminase each in the form of a powder separately or simultaneously with the food material. A case using any one of the above-described methods is encompassed by the bound and formed food production method of this invention.

As the food material to be used in this invention, any of proteinaceous food materials may be used. For example, not only so-called edible meats such as beef, pork, horse meat, mutton, goat meat, rabbit meat, and chicken, but also other food materials including various fish meats, shellfish, crustaceans such as shrimp and crab, mollusks such as squid and octopus, fish eggs such as salmon roe (ikura) and salmon roe (sujiko), and the like are usable. Further, a processed food such as cheese, pasta, and fish sausage are usable. Of course, the above-described food materials are not limitative.

The bound and formed food of this invention means a formed/reconstructed food obtained by binding a plurality of food pieces obtained by cutting the food material into an appropriate size (e.g.: those obtained by binding and forming meat pieces each having the size of 1 cm³) and a formed/reconstructed food obtained by binding a plurality of pieces of the material obtained without cutting (e.g. those obtained by binding and forming a plurality of pieces of small-size food material having the size of 1 cm³ or less such as salmon roe and shrimps) and does not include food produced from a paste-like food obtained by homogenizing treatment or a mincing treatment, such as a minced material and pasted material. However, a food obtained by binding and forming small pieces (not a paste) of a fish sausage or a sausage that is formed once is included in the bound and formed food of this invention.

The binding of food pieces or pieces of small food material is required to achieve a tensile strength of 80 g/cm² or more that is detected by a rheometer (manufactured by Fudo Kogyo KK). The tensile strength of 80 g/cm² or less is not suitable for practical use since such strength causes the pieces to be broken away during production of the bound and formed food or during cooking processing.

This invention is applicable to all the proteinaceous food materials as described above, and it is possible to produce the bound and formed food by using proteins from one origin in the case where the enzyme preparation of this invention that is prepared by using the beef-derived collagen is used for beef as the food material or in the case where the enzyme preparation of this invention that is prepared by using the pork-derived collagen is used for pork as the food material, thereby solving the problem in the meat processing industry demanding for food that is free from proteins of different origins and free from allergy labeling.

### Brief Description of the Drawings

Fig. 1 is a graph showing relationships between salts and a binding strength (Example 1).
Fig. 2 is a graph showing relationships between collagens and a binding strength (Example 2).
Fig. 3 is a graph showing relationships between collagens and a binding strength (Example 3).

### Best Mode for Carrying out the Invention

This invention will hereinafter be described based on examples. A technical scope of this invention is not limited to the examples.

### Example 1: Production of Bound and Formed Pork by Transglutaminase, Collagen, and Salts

A commercially available transglutaminase of Streptomyces mobaraensis-origin (specific activity: 1000 unit/g; "Activa" TG; manufactured by Ajinomoto Co., Inc.) was used as the transglutaminase. Streptomyces mobaraensis was called Streptoverticillium mobaraense before 1990.

As the collagen, Gelatin G Fine Powder, manufactured by Nitta Gelatin Inc. (acid-treated collagen having jelly strength of 256 g) was used.

In each of test samples, 1.2 g of the collagen (Gelatin G Fine Powder, manufactured by Nitta Gelatin Inc.) and 1.5 of a salt were weighed and dissolved into 10 ml of water. The transglutaminase was weighed, and 180 U of the transglutaminase was dissolved into 2 ml of water, followed by mixing with the formerly mentioned solution. The obtained mixed solution is the enzyme preparation of this invention.

The above-described conditions are the same as addition ratios when 1 part by weight of the enzyme preparation that is prepared by using 40 g of the collagen per 100 g of the enzyme preparation, 50 g of the salt per 100 g of the enzyme preparation, and 60 U of the transglutaminase per 1 g of the enzyme preparation is added to 100 parts by weight of a food material.

Each of the enzyme preparations obtained as described above was mixed with 300 g of small pieces (about 2 cm cube) of pork ham, and the mixture was filled into a casing tube having a folding width of 75 mm and left to stand at 5°C for 2 hours for progress of a reaction of the transglutaminase. After the standing, the mixture was stored in a refrigerator at -40°C for frozen storage until evaluation.

The added amounts of the collagen and the salt in each of test samples are equivalent to 0.4 g of the collagen and 0.5 g of the salt per 100 g of the food material. Also, the added amount of the transglutaminase is equivalent to 0.6 U per 1 g of the food material. Details are shown in Table 1.

**Table 1: Composition (added amounts per 300 g of meat)**

| | Sample 1 | Sample 2 | Sample 3 | Sample 4 | Sample 5 |
|---|---|---|---|---|---|
| Transglutaminase (0.6 U per 1 g of food material in each of test samples) | 180 U | 180 U | 180 U | 180 U | 180 U |
| Acid-treated collagen (0.4 g per 100 g of food material in each of test samples) | 1.2 g | 1.2 g | 1.2 g | 1.2 g | 1.2 g |
| Salt (0.5 g per 100 g of food material in each of test samples except where salt is not added) | salt was not added | 1.5 g of Sodium Chloride | 1.5 g of Potassium Chloride | 1.5 g of Calcium Chloride | 1.5 g of Magnesium Chloride |

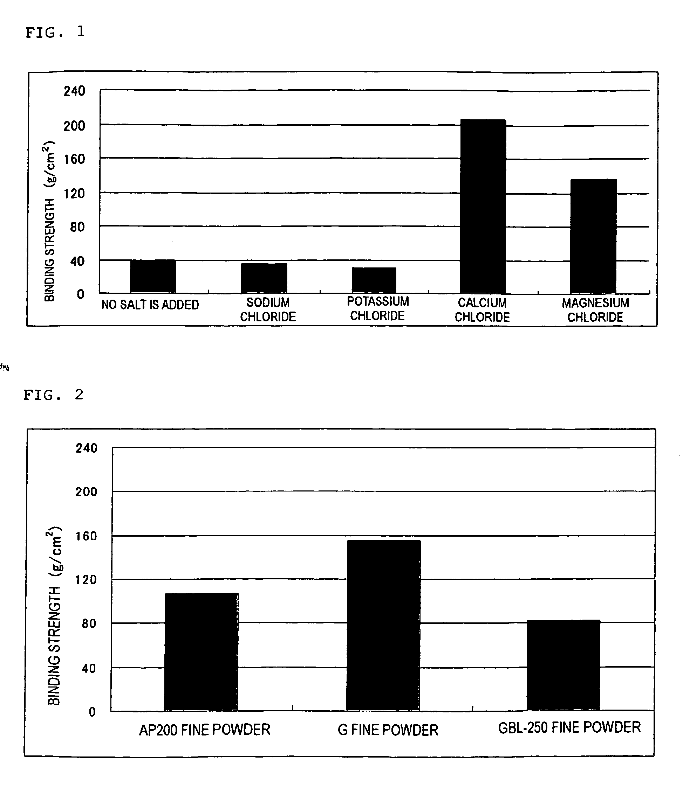

A slice having a thickness of 9 mm and a width of 25 mm was obtained by slicing each of the frozen bound porks, and, after thawing, a tensile strength of the slice in the raw condition was measured by using a rheometer (manufactured by Fudo Kogyo KK). Results are shown in Fig. 1. Though a tensile strength of 80 g/cm² is judged to be a practical binding strength in this evaluation method, the test sample in which calcium chloride or magnesium chloride was added achieved the binding force exceeding the reference value (80 g/cm²). Specifically, the test sample in which calcium chloride was added achieved the binding strength of 205.8 g/cm², and the test sample in which magnesium chloride was added achieved the binding strength of 136.1 g/cm². In contrast, the test sample in which no salt was added achieved the binding strength of 39.3 g/cm²; the test sample in which sodium chloride was added achieved the binding strength of 35.4 g/cm²; and the test sample in which potassium chloride was added achieved the binding strength of 30.0 g/cm², which are not practical binding strengths.

### Example 2: Type of Collagen and Influence thereof on Binding strength

1.2 g of each of collagens shown in Table 2 and 1.5 g of calcium chloride were dissolved into 10 ml of water. The transglutaminase was weighed, and 180 U of the transglutaminase was dissolved into 2 ml of water, followed by mixing with the formerly-mentioned solution of the collagen and calcium chloride. The obtained mixed solution is the enzyme preparation of this invention.

**Table 2: Characteristics of Collagen**

| Product Name | Extraction Method | Actual Measurement Value of Jelly Strength |
|---|---|---|
| AP200 Fine Powder (manufactured by Nitta Gelatin Inc.) | Acid Treatment | 199 g |
| G Fine Powder (manufactured by Nitta Gelatin Inc.) | Acid Treatment | 256 g |
| GBL250 Fine Powder (manufactured by Nitta Gelatin Inc.) | Alkali Treatment | 265 g |

Each of the enzyme preparations obtained as described above was mixed with 300 g of small pieces (about 2 cm cube) of pork ham, and the mixture was filled into a casing tube having a folding width of 75 mm and left to stand at 5°C for 2 hours for progress of a reaction of the transglutaminase. After the standing, the mixture was stored in a refrigerator at -40°C for frozen storage until evaluation. After that, tensile strengths were measured in the same manner as in Example 1. Results are shown in Fig. 2.

The added amounts of the collagen and the calcium chloride are equivalent to 0.4 g of the collagen and 0.5 g of the calcium chloride per 100 g of the food material. Also, the added amount of the transglutaminase is equivalent to 60 U per 100 g of the food material. Details are shown in Table 3.

**Table 3: Composition (added amounts per 300 g of meat)**

| | Sample 1 | Sample 2 | Sample 3 |
|---|---|---|---|
| Transglutaminase (0.6 U per 1 g of food material in each of test samples) | 180 U | 180 U | 180 U |
| Collagen in Use (0.4 g per 100 g of food material in each of test samples) | 1.2 g of AP200 Fine Powder | 1.2 g of G Fine Powder | 1.2 g of GBL250 Fine Powder |
| Calcium Chloride (0.5 g per 100 g of food material in each of test samples except where salt is not added) | 1.5 g | 1.5 g | 1.5 g |

As shown in Fig. 2, it was confirmed that it is possible to achieve the higher binding strength of 154.7 g/cm² by the use of the G powder having the high jelly strength.

Also, in view of the fact that the binding strength achieved by GBL-250 is 82.1 g/cm² and the binding strength achieved by AP200 Fine Powder is 106.5 g/cm², it was confirmed that the acid-treated collagen enables to achieve the stronger binding strength as compared to the alkali-treated collagen.

### Example 3: Type of Collagen and Influence thereof on Binding strength-2

1.2 g of each of collagens shown in Table 4 and 1.5 g of calcium chloride were dissolved into 10 ml of water. The transglutaminase was weighed, and 180 U of the transglutaminase was dissolved into 2 ml of water, followed by mixing with the formerly-mentioned solution of the collagen and calcium chloride. The obtained mixed solution is the enzyme preparation of this invention.

**Table 4: Characteristics of Collagen**

| Product Name | Raw Material | Actual Measurement Value of Gel Strength |
|---|---|---|
| T95-S (manufactured by BHJ) | Pig Skin | 50 g |
| WB1/20KD (manufactured by Prowico) | Pig Skin | 13 g |
| WB 1/40KD (manufactured by Prowico) | Pig Skin | 27 g |

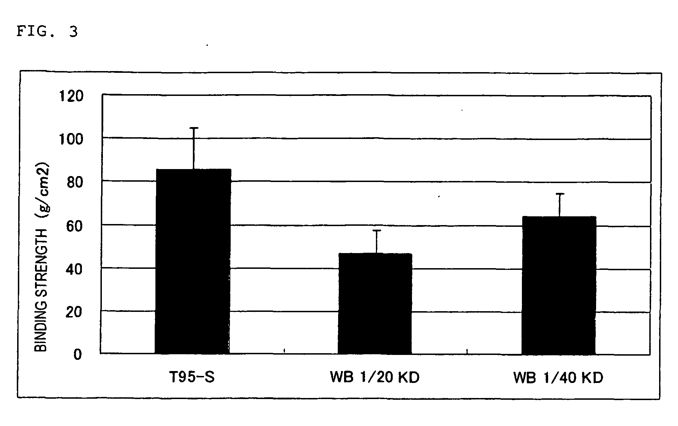

Each of the enzyme preparations obtained as described above was mixed with 300 g of small pieces (about 2 cm cube) of pork ham, and the mixture was filled into a casing tube having a folding width of 75 mm and left to stand at 5°C for 2 hours for progress of a reaction of the transglutaminase. After the standing, the mixture was stored in a refrigerator at -40°C for frozen storage until evaluation. After that, tensile strengths were measured in the same manner as in Example 1. Results are shown in Fig. 3. In order to measure a binding strength after heating, a slice having a thickness of 9 mm of each of the bound meats was heated at 240°C for 150 seconds for each side, and the strength was measured by sensory evaluation. Standards for sensory evaluation points are as shown in Table 5, and the evaluation was conducted by 3 sufficiently trained panelists. Results are shown in Table 6.

The added amounts of the collagen and the calcium chloride are equivalent to 0.4 g of the collagen and 0.5 g of the calcium chloride per 100 g of the food material. Also, the added amount of the transglutaminase is equivalent to 60 U per 100 g of the food material. Details are shown in Table 6.

**Table 5: Sensory Evaluation Standards**

| Sensory Evaluation Score | Binding State |
|---|---|
| 1 | Broken away into pieces after heating. |
| 2 | Pieces were bound, but large crack was observed. |
| 3 | No crack was observed, but detached easily when pulled. |
| 4 | Not detached easily when pulled. Practical strength is achieved. |
| 5 | Strong binding strength is achieved, so that a piece which is not on binding surface is deformed when pulled. |

**Table 6: Composition (added amounts per 300 g of meat) and Sensory Evaluation Results**

| | Sample 1 | Sample 2 | Sample 3 |
|---|---|---|---|
| Collagen in Use (0.4 g per 100 g of food material in each of test samples) | 1.2 g of T95-S (pig skin-derived; gel strength: 50 g) | 1.2 g of WB 1/20KD (pig skin-derived; gel strength: 13 g) | 1.2 g of WB 1/40KD (pig skin-derived; gel strength: 27 g) |
| Calcium Chloride (0.5 g per 100 g of food material in each of test samples except where salt is not added) | 1.5 g | 1.5 g | 1.5 g |
| Transglutaminase (0.6 U per 1 g of food material in each of test samples) | 180 U | 180 U | 180 U |
| Evaluation Score after heating | 4 | 2 | 3 |

As shown in Fig. 3, it was confirmed that it is possible to achieve the higher binding strength of 85.5 g/cm² by the use of the collagen having the high jelly strength.

### Industrial Applicability

This invention is usable for binding a food material. Also, the enzyme preparation of this invention imparts not only the satisfactory binding strength but also favorable texture to an ultimately obtained bound and formed food.

With the use of the enzyme preparation and the production method for bound and formed food of this invention, it is possible to satisfactorily bind and form the food material such as meat pieces without using a fish-derived collagen. Therefore, this invention is a highly practical and excellent technology that is much expected in the processed food industry.

## Claims

1. An enzyme preparation comprising as active ingredients a transglutaminase derived from a microorganism, animal collagen not including fish and shellfish collagen and calcium chloride or magnesium chloride, wherein an amount of calcium chloride or magnesium chloride in the enzyme preparation is 0. 007 to 0.03 g per 1 unit of the transglutaminase in the enzyme preparation, and an amount of the collagen in the enzyme preparation is 0.002 to 0.03 g per 1 unit of the transglutaminase in the enzyme preparation.

2. The enzyme preparation according to claim 1, wherein a gel strength of the collagen comprised in the enzyme preparation is 40 g or more.

3. The enzyme preparation according to claim 1, wherein a jelly strength of the collagen comprised in the enzyme preparation is 150 g or more.

4. The enzyme preparation according to claim 3, wherein the collagen is an acid-treated collagen.

5. A method for producing a bound and formed food, wherein the enzyme preparation according to claims 1 to 4 is (1) dissolved into water or a liquid to be added to a food material or (2) added directly to a food material.

6. Amethod for producing a bound and formed food, wherein 10 to 100 units of a transglutaminase derived from a microorganism, 0.1 to 2 g of animal collagen not including fish and shellfish collagen and 0.4 to 2 g of calcium chloride or magnesium chloride per 100 g of the food material are (1) dissolved into water or a liquid to be added to a food material or (2) added directly to a food material.

7. The method according to claim 6, wherein a gel strength of the collagen comprised in the enzyme preparation is 40 g or more.

8. The method according to claim 6, wherein a jelly strength of the collagen comprised in the enzyme preparation is 150 g or more.

9. The bound and formed food production method according to claim 8, wherein the collagen is an acid-treated collagen.

## Patentansprüche

1. Enzymzubereitung, die als aktive Bestandteile eine von einem Microorganismus abgeleitete Transglutaminase, tierisches Collagen außer Fisch- und Schalentier-Collagen und Calciumchlorid oder Magnesiumchlorid enthält, wobei eine Menge des Calciumchlorids oder Magnesiumchlorids in der Enzymzubereitung 0,007 bis 0,03 g pro 1 Einheit der Transglutaminase in der Enzymzubereitung ist und eine Menge des Collagens in der Enzymzubereitung 0,002 bis 0,03 g pro 1 Einheit der Transglutaminase in der Enzymzubereitung ist.

2. Enzymzubereitung nach Anspruch 1, wobei die Gelstärke des in der Enzymzubereitung enthaltenen Collagens 40 g oder mehr ist.

3. Enzymzubereitung nach Anspruch 1, wobei die Geleestärke des in der Enzymzubereitung enthaltenen Collagens 150 g oder mehr ist.

4. Enzymzubereitung nach Anspruch 3, wobei das Collagen ein säurebehandeltes Collagen ist.

5. Verfahren zum Herstellen eines gebundenen und geformten Nahrungsmittels, wobei die Enzymzubereitung nach den Ansprüchen 1 bis 4 (1) in Wasser oder einer Flüssigkeit, das/die zu einem Nahrungsmittelmaterial gegeben werden soll, gelöst wird oder (2) direkt zu dem Nahrungsmittelmaterial gegeben wird.

6. Verfahren zum Herstellen eines gebundenen und geformten Nahrungsmittels, wobei 10 bis 100 Einheiten einer von einem Mikroorganismus abgeleiteten Transglutaminase, 0,1 bis 2 g tierisches Collagen außer Fisch- und Schalentier-Collagen und 0,4 bis 2 g Calciumchlorid oder Magnesiumchlorid pro 100 g des Nahrungsmittelmaterials (1) in Wasser oder einer Flüssigkeit, das/die zu dem Nahrungsmittelmaterial gegeben werden soll, gelöst werden oder (2) direkt zu dem Nahrungsmittelmaterial gegeben werden.

7. Verfahren nach Anspruch 6, wobei eine Gelstärke des in der Enzymzubereitung enthaltenen Collagens 40 g oder mehr ist.

8. Verfahren nach Anspruch 6, wobei die Geleestärke des in der Enzymzubereitung enthaltenen Collagens 150 g oder mehr ist.

9. Verfahren zum Herstellen von gebundenem und geformtem Nahrungsmittel nach Anspruch 8, wobei das Collagen ein säurebehandeltes Collagen ist.

## Revendications

1. Préparation enzymatique comprenant comme ingrédients actifs une transglutaminase dérivée d'un microorganisme, du collagène animal qui ne comprend pas de collagènes de poisson et de crustacés; et du chlorure de calcium ou du chlorure de magnésium, dans laquelle une quantité de chlorure de calcium ou de chlorure de magnésium dans la préparation enzymatique est de 0,007 à 0,03 g par unité de transglutaminase dans la préparation enzymatique, et une quantité de collagène dans la préparation enzymatique est de 0,002 à 0,03 g par unité de transglutaminase dans la préparation enzymatique.

2. Préparation enzymatique selon la revendication 1, dans laquelle la rigidité de gel du collagène, compris dans la préparation enzymatique, est de 40 g ou plus.

3. Préparation enzymatique selon la revendication 1, dans laquelle la solidité de gel du collagène, compris dans la préparation enzymatique, est de 150 g ou plus.

4. Préparation enzymatique selon la revendication 3, dans laquelle le collagène est un collagène traité à l'acide.

5. Procédé pour produire un aliment formé et lié, dans lequel la préparation enzymatique selon les revendications 1 à 4 est (1) dissoute dans l'eau ou un liquide à ajouter à un produit alimentaire ou (2) ajoutée directement à un produit alimentaire.

6. Procédé pour produire un aliment formé et lié, dans lequel 10 à 100 unités d'une transglutaminase dérivée d'un microorganisme, 0,1 à 2 g de collagène animal qui ne comprend pas de collagènes de poisson et de crustacés ; et 0,4 à 2 g de chlorure de calcium ou de chlorure de magnésium pour 100 g de produit alimentaire sont (1) dissous dans l'eau ou un liquide à ajouter à un produit alimentaire ou (2) ajoutés directement à un produit alimentaire.

7. Procédé selon la revendication 6, dans lequel la rigidité de gel du collagène, compris dans la préparation enzymatique, est de 40 g ou plus.

8. Procédé selon la revendication 6, dans lequel la solidité de gel du collagène, compris dans la préparation enzymatique, est de 150 g ou plus.

9. Procédé de production d'un aliment formé et lié selon la revendication 8, dans lequel le collagène est un collagène traité à l'acide.
